(19) Europäisches Patentamt
European Patent Office
Office européen des brevets

(11) EP 1 310 235 A2

(12) **EUROPÄISCHE PATENTANMELDUNG**

(43) Veröffentlichungstag:
**14.05.2003 Patentblatt 2003/20**

(51) Int Cl.[7]: **A61K 7/42**

(21) Anmeldenummer: **02023510.7**

(22) Anmeldetag: **22.10.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **09.11.2001 DE 10155966**

(71) Anmelder: **Beiersdorf AG**
**20245 Hamburg (DE)**

(72) Erfinder:
- **Göppel, Anja**
  **22527 Hamburg (DE)**
- **Koopmann, Sabine**
  **20253 Hamburg (DE)**
- **Schulz, Jens, Dr.**
  **22869 Schenefeld (DE)**
- **Grotelüschen, Birgit**
  **22459 Hamburg (DE)**

(54) **Emulgatorfreie kosmetische und dermatologische Lichtschutzformulierungen mit einem Gehalt an Hydroxybenphenonen**

(57) Pickering-Emulsionen, welche feindisperse Systeme vom Typ Wasser-in-Öl oder Öl-in-Wasser darstellen, enthaltend

(1) eine Ölphase,
(2) eine Wasserphase,
(3) mindestens einen Typ mikrofeiner Partikel, die

a) eine mittlere Partikelgröße von weniger als 200 nm haben, die
b) sowohl hydrophile als auch lipophile Eigenschaften zeigen, welche also amphiphilen Charakter besitzen und sowohl in Wasser als auch in Öl dispergierbar sind und die
c) gegebenenfalls oberflächlich beschichtet sind,

(4) mindestens ein Hydroxybenzophenon und
(5) höchstens 1 Gew.-% eines oder mehrerer Emulgatoren.

EP 1 310 235 A2

## Beschreibung

Die vorliegende Erfindung betrifft emulgatorfreie feindisperse Systeme vom Typ Öl-in-Wasser und Wasser-in-Öl, bevorzugt als kosmetische oder dermatologische Lichtschutzzubereitungen.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:

- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Verbindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar.

Kosmetische Zubereitungen werden im wesentlichen zur Hautpflege benutzt. Hautpflege im kosmetischen Sinn ist in erster Linie, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Kosmetische Zubereitungen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Medizinische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder

auf Überempfindlichkeit des Anwenders beruhende Reaktionen hervorrufen.

So ist beispielsweise bekannt, daß bestimmte Lichtdermatosen durch gewisse Emulgatoren, aber auch durch verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch Zusammenfließen der dispersen Phasen verhindert wird. Von wesentlicher Bedeutung sind dabei nicht die chemischen, sondern die Oberflächeneigenschaften der Feststoffpartikel.

Pickering stellte um 1910 erstmals Paraffin-Wasser-Emulsionen her, die nur durch den Zusatz verschiedener Feststoffe wie basisches Kupfersulfat, basisches Eisensulfat oder andere Metallsulfate stabilisiert wurden. Diese Art von Emulsionen wird daher auch als Pickering-Emulsion bezeichnet.

Sozusagen die Urformen von Pickering-Emulsionen tauchten zunächst als unerwünschte Nebeneffekte bei unterschiedlichen technischen Prozessen auf, wie z. B. bei der sekundären Erdölförderung, der Extraktion von Bitumen aus Teersand und anderen Trennungsverfahren, an denen zwei nicht miteinander mischbare Flüssigkeiten und feine, dispergierte Feststoffpartikel beteiligt sind. Im allgemeinen handelt es sich hierbei um W/O-Emulsionen, die durch mineralische Feststoffe stabilisiert werden. Dementsprechend stand die Untersuchung entsprechender Systeme, wie z. B. der Systeme Öl-Wasser-Ruß oder Öl-Wasser-Schieferstaub zunächst im Mittelpunkt der Forschungsaktivitäten.

Grundlegende Untersuchungen haben dabei gezeigt, daß ein Charakteristikum für eine Pickering-Emulsion ist, daß die Feststoffpartikel an der Grenzfläche zwischen den beiden flüssigen Phasen angeordnet sind und dort sozusagen eine mechanische Barriere gegen die Vereinigung der Flüssigkeitströpfen bilden.

Eine relativ neue technische Entwicklung ist es, Pickering-Emulsionen als Grundlage für kosmetische oder dermatologische Zubereitungen zu verwenden.

Eine Möglichkeit, eine Feststoffstabilisierung im Sinne einer Pickering-Emulsion in einer kosmetischen oder dermatologischen Zubereitung vorzunehmen, ist nach May-Alert (*Pharmazie in unserer Zeit, 15. Jahrg. 1986, Nr. 1, 1-7*) beispielsweise, Emulgatorgemische zu verwenden, die sowohl anionische als auch kationische Tenside enthalten. Da beim Zusammengeben von Anion- und Kationtensiden immer unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung erreichen.

Darüber hinaus beschreibt die Europäische Offenlegungsschrift 0 686 391 Emulsionen vom Typ Wasser-in-Öl, die frei von oberflächenaktiven Substanzen sind und nur durch Feststoffe stabilisiert werden. Zur Stabilisierung werden hier sphärische Polyalkylsilsesquioxan-Partikel eingesetzt, die einen Durchmesser von 100 nm bis zu 20 µm haben. Diese Emulsionen können nach dem oben gesagten als Pickering-Emulsionen bezeichnet werden.

Pickering-Emulsionen werden durch den Einsatz von geeigneten Feststoffen bzw. Pigmenten stabilisiert. Allerdings können Feststoffe bei der Anwendung entsprechender Zubereitungen auf der Haut einen trockenen und zum Teil stumpfen Eindruck hinterlassen. Bereits Zubereitungen mit einem Pigmentgehalt von 1 Gew.-% erzeugen nach ihrer Anwendung ein stumpfes Gefühl auf der Haut, das mit höheren Pigmentkonzentrationen noch zunimmt. Im Einzelfall können daher pigmenthaltige Zubereitungen sogar nicht vermarktungsfähig sein, da sie vom Verbraucher nicht akzeptiert bzw. negativ beurteilt werden.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich beispielsweise um Derivate des 3-Benzylidencamphers, der 4-Aminobenzoesäure, der Zimtsäure, der Salicylsäure, des Benzophenons sowie des s-Triazins handelt.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u. a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut

"altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von bräunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Es ist daher von grundsätzlicher Wichtigkeit, daß kosmetische und dermatologische Lichtschutzzubereitungen sowohl gegen UV-B- als auch gegen UV-A-Strahlung ausreichenden Schutz bieten.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen.

Die Einsatzkonzentration bekannter als Feststoff vorliegender Lichtschutzfiltersubstanzen ist allerdings häufig - gerade in Kombination mit anderen zu lösenden Substanzen - begrenzt. Es bereitet daher gewisse formulierungstechnische Schwierigkeiten, höhere Lichtschutzfaktoren bzw. UV-A-Schutzleistung zu erzielen.

Der Sonnenbrand bzw. das Lichterythem sind die akuten Erscheinungsformen der Lichteinwirkung. Neben den bereits beschriebenen Wirkungen der UV-Strahlen kommt es in der Nachreaktion der Haut ferner zu einer verminderten Sebumproduktion und einem Austrocknen der Haut. Zur Linderung und zur Pflege dieser Phänomene kennt der Stand der Technik Produkte, welche nach dem Sonnenbaden angewendet werden und üblicherweise spezielle Wirkstoffe enthalten, wie beispielsweise

- Rückfettungs- und Feuchthaltemittel,
- entzündungslindernde und kühlende Stoffe,
- lokal anaestesierende Stoffe und/oder
- desinfizierende Stoffe, um mögliche Hautinfektionen zu verhindern.

Diese sogenannten Aftersun- oder Après-soleil-Präparate sind dazu bestimmt, die Haut nach dem Sonnenbad zu kühlen und ihr Feuchthaltevermögen zu verbessern, wobei die Vermittlung des Kühleffektes eine zentrale Rolle spielt. Allerdings mangelt es dem Stand der Technik an Produkten, welche die Haut bereits während der UV-Einstrahlung vor dem Austrocknen schützen und sie ausreichend pflegen.

Aufgabe war daher, den Nachteilen des Standes der Technik Abhilfe zu schaffen. Insbesondere sollten Zubereitungen zur Verfügung gestellt werden, welche auf der Haut keinen trockenen oder stumpfen Eindruck hinterlassen.

Weiterhin war es eine Aufgabe der Erfindung, kosmetische und dermatologische Grundlagen für kosmetische und dermatologische Zubereitungen zu entwickeln, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen oder Deodorantien, aber auch ausgesprochen medizinisch-pharmazeutische Darreichungsformen geschaffen werden, zum Beispiel Zubereitungen gegen Akne und andere Hauterscheinungen.

Es war erstaunlich und für den Fachmann in keiner Weise vorauszusehen, daß Pickering-Emulsionen, welche feindisperse Systeme vom Typ Wasser-in-Öl oder Öl-in-Wasser darstellen, enthaltend

(1) eine Ölphase,
(2) eine Wasserphase,
(3) mindestens einen Typ mikrofeiner Partikel, die

  a) eine mittlere Partikelgröße von weniger als 200 nm haben, die
  b) sowohl hydrophile als auch lipophile Eigenschaften zeigen, welche also amphiphilen Charakter besitzen und sowohl in Wasser als auch in Öl dispergierbar sind und die
  c) gegebenenfalls oberflächlich beschichtet sind,

(4) mindestens ein Hydroxybenzophenon und
(5) höchstens 1 Gew.-% eines oder mehrerer Emulgatoren

den Nachteilen des Standes der Technik abhelfen.

Es ist erfindungsgemäß besonders vorteilhaft, wenn die Zubereitungen deutlich weniger als 1 Gew.-% eines oder mehrerer Emulgatoren enthalten bzw. sogar gänzlich emulgatorfrei sind.

Die erfindungsgemäßen Zubereitungen sind in jeglicher Hinsicht überaus befriedigende Präparate, die erstaunlicherweise hervorragende kosmetische Eigenschaften zeigen, auf der Haut keinen trockenen oder stumpfen Eindruck hinterlassen und sich durch eine ausgezeichnete Hautverträglichkeit auszeichnen.

Zwar kennt der Stand der Technik neben Pickering-Emulsionen emulgatorfreie, feindisperse kosmetische oder dermatologische Zubereitungen, die im allgemeinen als Hydrodispersionen bezeichnet werden und welche Dispersionen einer flüssigen, halbfesten oder festen inneren (diskontinuierlichen) Lipidphase in einer äußeren wäßrigen (kontinuierlichen) Phase darstellen. Allerdings konnte der Stand der Technik nicht den Weg zur vorliegenden Erfindung weisen.

Bei Hydrodispersionen einer flüssigen Lipidphase in einer äußeren wäßrigen Phase kann die Stabilität beispielsweise dadurch gewährleistet werden, daß in der wäßrigen Phase ein Gelgerüst aufgebaut wird, in welchem die Lipidtröpfchen stabil suspendiert sind.

Die Deutsche Offenlegungsschrift 44 25 268 beschreibt stabile feindisperse, emulgatorfreie kosmetische oder dermatologische Zubereitungen vom Typ Öl-in-Wasser, die neben einer Öl- und einer Wasserphase einen oder mehrere Verdicker aus der Gruppe der Acrylsäurepolymere, Polysaccharide und deren Alkylether enthalten, wobei für diese Verdicker eine Grenzflächenspannungserniedrigung nicht meßbar sein darf.

Basierend auf ähnlichen Hydrodispersionen werden in der Deutschen Offenlegungsschrift 43 03 983 kosmetische oder dermatologische Lichtschutzformulierungen offenbart, die im wesentlichen frei von Emulgatoren sind, wobei in die Lipidphase der Hydrodispersion anorganische Mikropigmente eingearbeitet sind, die als UV-Filtersubstanzen dienen.

O/W-Pickering-Emulsionen im Sinne der vorliegenden Erfindung hingegen sind erhältlich, indem man erfindungsgemäße, für die Herstellung von O/W-Pickering-Emulsionen geeignete amphiphile Partikel zunächst in der Wasserphase dispergiert und die Wasserphase anschließend mit der Fettphase vereinigt. Erfindungsgemäße W/O-Pickering-Emulsionen sind dagegen durch Dispergieren von erfindungsgemäßen, für die Herstellung von W/O-Pickering-Emulsionen geeigneten amphiphilen Partikeln in der Fettphase erhältlich.

Die UV-Schutzleistung von Sonnenschutzmittel bzw. der ihnen zugrunde liegenden UV-Filter wird in der Regel in biologischen Wirksamkeitsprüfungen unter standardisierten Bedingungen bestimmt. Mit "UV-Schutzleistung" ist im Sinne der vorliegenden Erfindung sowohl die Schutzleistung gegenüber UV-A-Strahlung als auch gegenüber UV-B-Strahlung gemeint.

Ein Maß für die UV-Schutzleistung stellen im Sinne der vorliegenden Erfindung beispielsweise der Lichtschutzfaktor (LSF bzw. SPF) oder auch IPD-Werte und dergleichen dar.

Der Lichtschutzfaktor (LSF, oft auch SPF (sun protection factor) genannt) gibt die Verlängerung der Sonnenbestrahlung an, die durch Verwendung des Sonnenschutzmittels ermöglicht wird. Er ist der Quotient aus Erythemschwellenzeit mit Sonnenschutzmittel und Erythemschwellenzeit ohne Sonnenschutzmittel.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich von 320 bis 360 nm absorbieren.

Hydroxybenzophenone zeichnen sich durch die folgende Strukturformel aus:

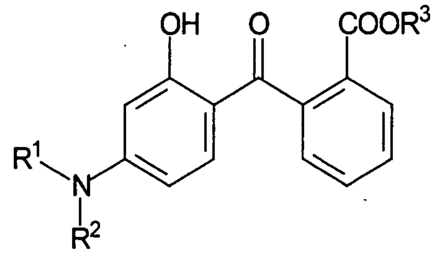

worin

- $R^1$ und $R^2$ unabhängig voneinander Wasserstoff, $C_1$-$C_{20}$-Alkyl, $C_3$-$C_{10}$-Cycloalkyl oder $C_3$-$C_{10}$-Cycloalkenyl be-

deuten, wobei die Substituenten $R^1$ und $R^2$ gemeinsam mit dem Stickstoffatom, an das sie gebunden sind, einen 5- oder 6-Ring bilden können und

- $R^3$ einen $C_1$-$C_{20}$-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher durch die chemische Strukturformel

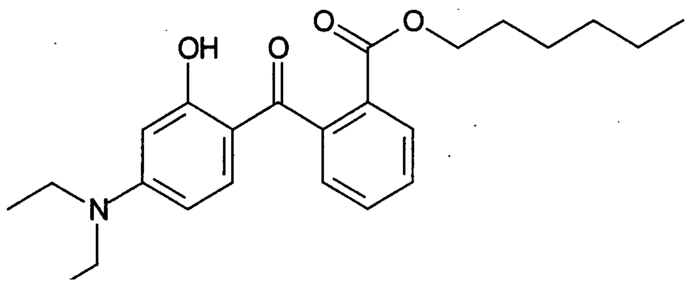

gekennzeichnet ist.

Erfindungsgemäß enthalten kosmetische oder dermatologische Zubereitungen 0,1 bis 20 Gew.-%, vorteilhaft 0,1 bis 15 Gew.-%, ganz besonders bevorzugt 0,1 bis 10 Gew.-% eines oder mehrerer Hydroxybenzophenone.

**Hilfsstoffe:**

Es ist ferner vorteilhaft, wenngleich nicht zwingend, wenn die erfindungsgemäßen feststoffstabilisierten Emulsionen neben Hydroxybenzophenon und/oder seinen Derivaten, weitere Hilfsstoffe enthalten, die zur Verminderung oder Verhinderung eines stumpfen oder trockenen Hautgefühls nach deren Auftragen beitragen können, wobei der hauptsächliche Zweck dieser Stoffe ein anderer sein kann.

Vorzugsweise werden diese Stoffe beispielsweise gewählt aus der Gruppe der polymeren Moisturizer. Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren.

Es ist insbesondere von Vorteil, die polymeren Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Letzteres zeichnet sich durch die folgenden Strukturelemente aus:

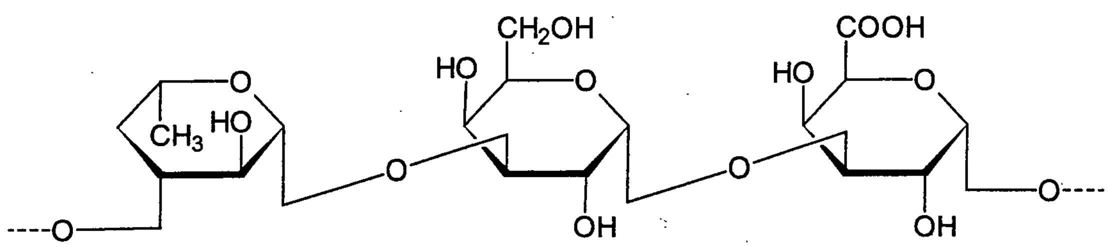

Hyaluronsäure zeichnet sich durch die Struktur

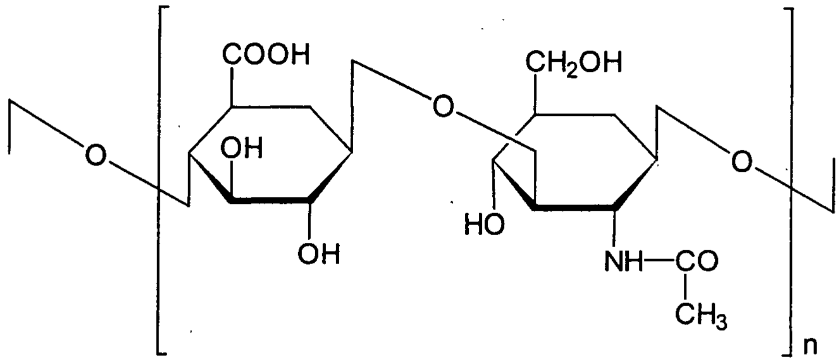

Es ist insbesondere bevorzugt, Hyaluronsäuren mit Molekulargewichten zwischen 30.000 und 8.000.000 zu wählen, insbesondere solche mit Molekulargewichten zwischen 500.000 und 1.500.000.

Chitosan ist gekennzeichnet durch folgende Strukturformel:

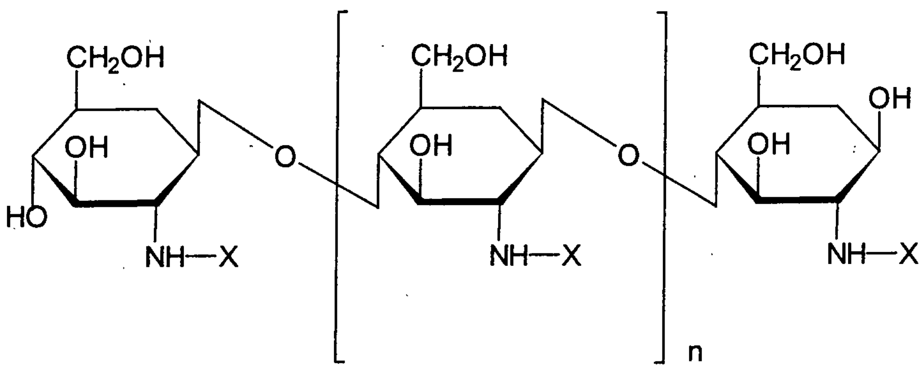

dabei nimmt n Werte bis zu ca. 10.000 an, X stellt entweder den Acetylrest oder Wasserstoff dar. Chitosan entsteht durch Deacetylierung und teilweise Depolymerisation (Hydrolyse) von Chitin, welches durch die Strukturformel

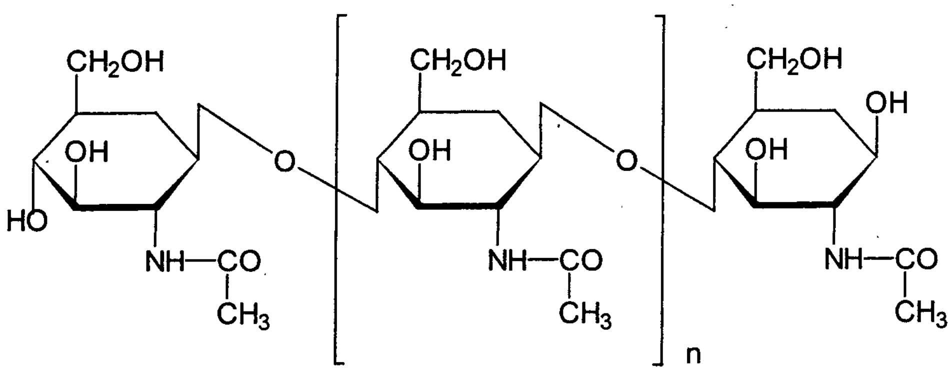

gekennzeichnet ist. Chitin ist wesentlicher Bestandteil des Ektoskeletts der Gliederfüßer (z. B. Insekten, Krebse, Spinnen) und wird auch in Stützgeweben anderer Organismen (z. B. Weichtiere, Algen, Pilze) gefunden.

Chitosan ist ein in der Haarpflege bekannter Rohstoff. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, "Lexikon der Hilfsstoffe für Pharmazie, Kosmetik und angrenzende Gebiete", 3. Auflage 1989, Editio Cantor, Aulendorf, S. 293, Stichwort "Chitosan".

Erfindungsgemäß bevorzugt sind Chitosane mit einem Deacetylierungsgrad größer 25 %, insbesondere größer 55 bis 99 % [bestimmt mittels $^1$H-NMR].

Es ist von Vorteil, Chitosane mit Molekulargewichten zwischen 3.000 und 2.000.000 zu wählen, insbesondere

solche mit Molekulargewichten zwischen 100.000 und 1.000.000. [bestimmt mittels Gelpermetionschromatographie].

Die Einarbeitung des Chitosans erfolgt in der Regel dadurch, daß eine Suspension von Chitosan, insbesondere von mikronisiertem Chitosan, in Wasser mit Hilfe von organischen oder anorganischen Säuren auf einen pH-Wert von ca. 3,5 - 5,5 gestellt, wobei - in der Regel durch Rühren - eine Lösung des Chitosans erhalten wird. Eine so erhältliche klare Lösung von 2 Gew.-% Chitosan in 1,2 % Milchsäure (90 %ige wäßrige Lösung) beispielsweise hat eine Viskosität nach Viskotester-VT02 (Haake), die im Bereich von 100 bis 10.000 mPa·s, vorzugsweise von 200 bis 5.000 mPa·s liegt.

Weiterhin vorteilhaft werden die Hilfsstoffe beispielsweise gewählt aus der Gruppe der Filmbildner. Filmbildner sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen eine Schutzfunktion hat.

Es ist insbesondere von Vorteil, die Filmbildner aus der Gruppe der Polymere auf Basis von Polyvinylpyrrolidon (PVP)

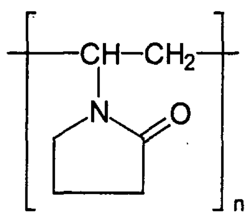

zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000.

Es ist ferner vorteilhaft, die Hilfsstoffe, die zur Verminderung oder Verhinderung eines stumpfen oder trockenen Hautgefühls nach Auftragen der erfindungsgemäßen Zubereitungen beitragen können, aus der Gruppe der Cyclodextrine zu wählen.

Cyclodextrine (Cycloamylosen, Cycloglucane) werden in kosmetischen und pharmazeutischen Zubereitungen oftmals zur "molekularen Verkapselung" verwendet, also als schützende Umhüllung empfindlicher Moleküle. Es handelt sich dabei um 6, 7, 8 oder noch mehr α-1,4-verknüpfte Glucoseeinheiten, wobei die Cyclohexaamylose (α-Cyclodextrin) sich durch die Struktur

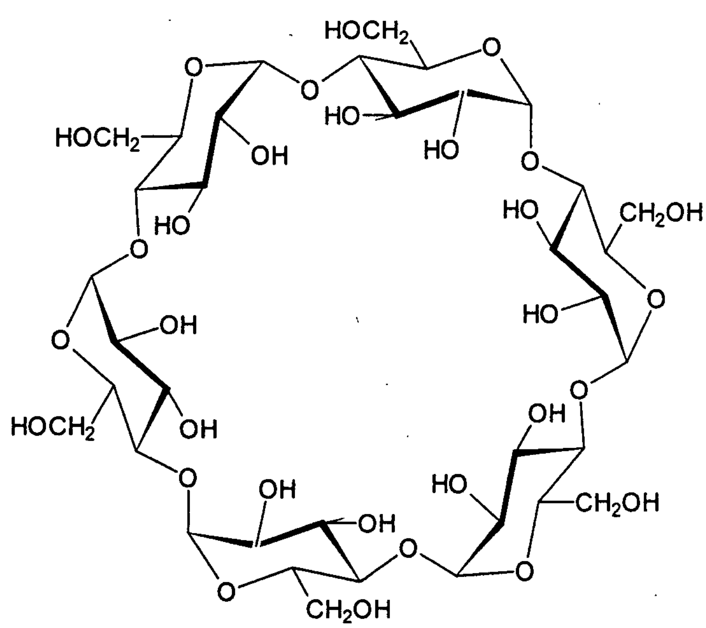

auszeichnet. Die Cycloheptaamylose (β-Cyclodextrin) zeichnet sich durch die Struktur

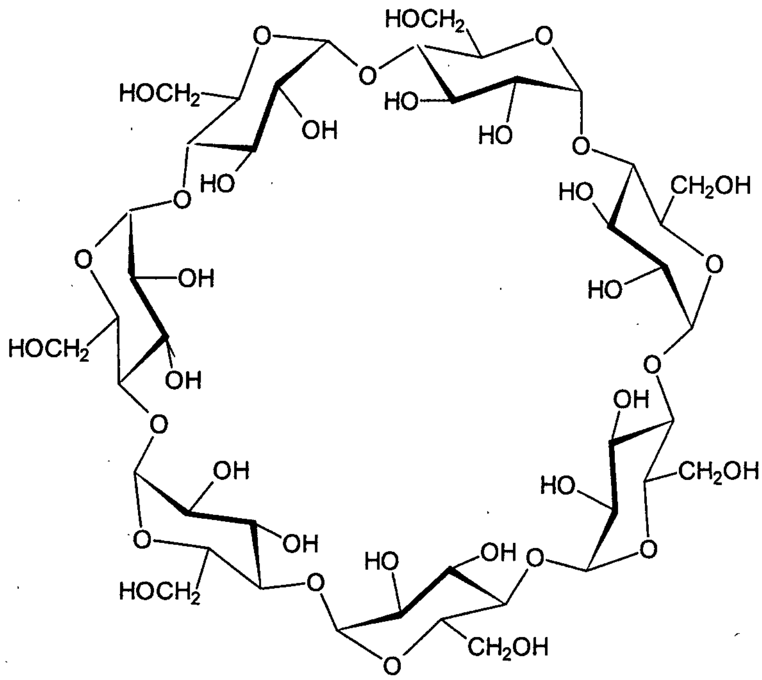

aus. Die Cyclooctaamylose (γ-Cyclodextrin) zeichnet sich durch die Struktur

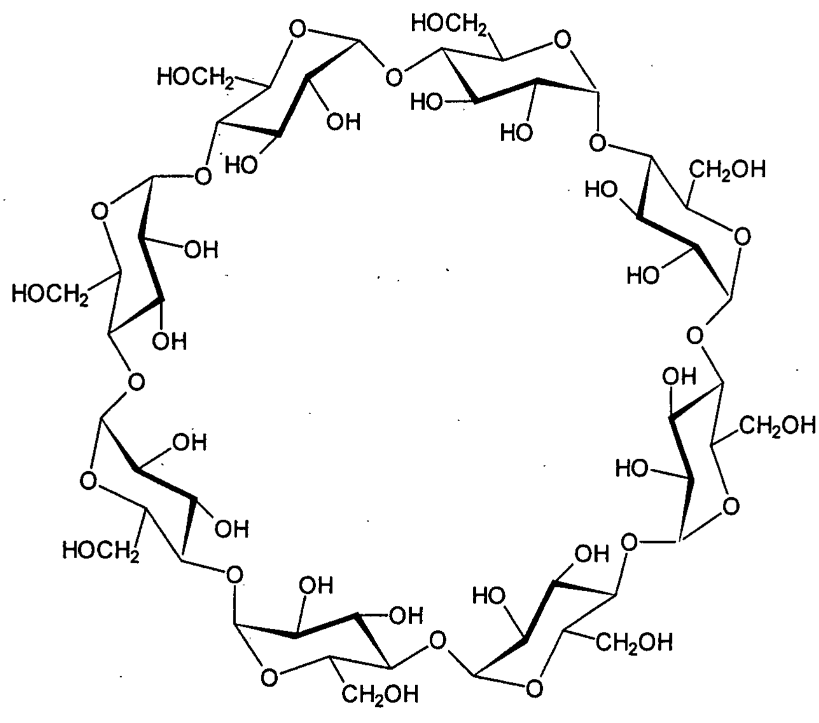

aus. Die Cycloenneaamylose (δ-Cyclodextrin) zeichnet sich durch die Struktur

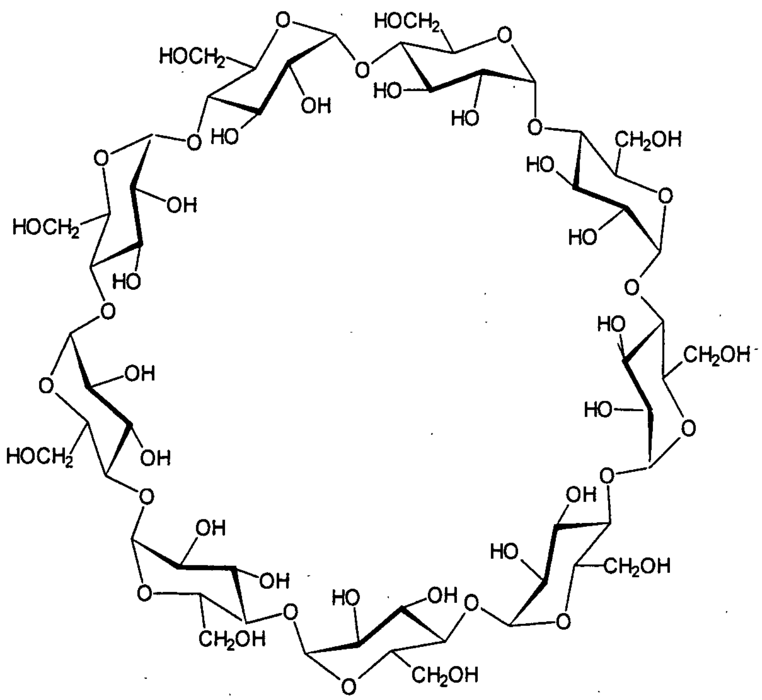

aus.

Besonders bevorzugt sind β-Cyclodextrin und γ-Cyclodextrin, beispielsweise die unter den Handelsbezeichnungen Beta W7 Pharma und Gamma W8 bei der Wacker Chemie erhältlichen.

Vorteilhaft im Sinne der vorliegenden Erfindung ist ferner insbesondere die Verwendung mehrerer verschiedener Hilfsstoffe, die zur Verminderung oder Verhinderung eines stumpfen oder trockenen Hautgefühls nach Auftragen der erfindungsgemäßen Zubereitungen beitragen können, aus einer oder mehrerer der obengenannten Gruppen.

Die kosmetischen Eigenschaften der erfindungsgemäßen Pickering-Emulsionen können noch zusätzlich beispielsweise dadurch verbessert werden, daß in der Ölphase Öle eingesetzt werden, welche eine Viskosität haben, die kleiner als 30 mPa·s, insbesondere kleiner als 20 mPa·s ist (bestimmt mit einem Rheometer der Firma Contraves (Rheomat 108E) bei einem Schergefälle von 500/s und einer Temperatur von 25 °C).

Es ist vorteilhaft, die dünnflüssigen Öle, die zur Verminderung oder Verhinderung eines stumpfen oder trokkenen Hautgefühls nach Auftragen der erfindungsgemäßen Zubereitungen beitragen können, aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cocoglyceride (z. B. Myritol® 331 von Henkel), $C_{12-13}$-Alkyllactat, Di-$C_{12-13}$-Alkyltartrat, Triisostearin, Dipentaerythrityl Hexacaprylat/Hexacaprat, Propylenglykol monoisostearat, Tricaprylin, Dimethylisosorbid, Stearylheptanoat, Cyclomethicon, Dimethicon, Phenyltrimethicon, $C_{12-15}$ -Alkylbenzoat zu wählen. Es ist insbesondere vorteilhaft, wenn die Ölphase der erfindungsgemäßen Formulierungen einen Gehalt an $C_{12-15}$-Alkylbenzoat aufweist oder vollständig aus diesem besteht.

Vorzugsweise machen die dünnflüssigen Öle mindestens 0,5 Gew.-% der Ölphase aus. Es ist aber gegebenenfalls auch von Vorteil, wenn die gesamte Ölphase aus dünnflüssigen Ölen besteht.

Die Ölphase der erfindungsgemäßen Formulierungen kann außerdem vorteilhaft gewählt werden aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/ oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Corapan TQ*).

Auch beliebige Abmischungen solcher Ölkomponenten sind vorteilhaft im Sinne der vorliegenden Erfindung einzusetzen.

## Mikrofeine Partikel:

Der amphiphile Charakter der erfindungsgemäßen mikrofeinen Partikel zeigt sich beispielsweise darin, daß diese sowohl in Wasser als auch in Öl dispergierbar sind.

Es ist vorteilhaft, den mittleren Partikeldurchmesser der verwendeten Partikel zwischen 1 nm und 200 nm, besonders vorteilhaft zwischen 5 nm und 100 nm zu wählen.

Es ist ferner vorteilhaft, die Konzentration aller erfindungsgemäßen amphiphilen Partikel größer als 0,1 Gew.-%, besonders vorteilhaft zwischen 0,1 Gew.-% und 30 Gew.-%, bezogen auf das Gesamtgewicht der Zubereitungen, zu wählen.

Vorteilhaft im Sinne der vorliegenden Erfindung sind alle Partikel, die geeignet sind, Pickering-W/O-Emulsionen bzw. Pickering-O/W-Emulsionen zu stabilisieren. Im wesentlichen unerheblich für die vorliegende Erfindung ist es, in welcher der gegebenenfalls natürlich vorkommenden Modifikationen die Partikel vorliegen.

Vorzugsweise werden zur Stabilisierung der Pickering-Emulsionen unbehandelte, nahezu reine Pigmentpartikel verwendet, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Pickering-Emulsionen im Sinne der vorliegenden Erfindung werden ebenfalls vorteilhaft durch anorganische Pigmente stabilisiert, die oberflächlich wasserabweisend behandelt ("gecoatet") sind, wobei gleichzeitig der amphiphile Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Bevorzugte anorganische Pigmente sind Metalloxide und/oder andere in Wasser schwerlösliche oder unlösliche Metallverbindungen, insbesondere Oxide des Titans ($TiO_2$), Zinks ($ZnO$), Eisens (z. B. $Fe_2O_3$), Zirkoniums ($ZrO_2$), Siliciums ($SiO_2$), Mangans (z. B. $MnO$), Aluminiums ($Al_2O_3$), Cers (z. B. $Ce_2O_3$), Mischoxide der entsprechenden Metalle sowie Abmischungen aus solchen Oxiden, sowie das Sulfat des Bariums ($BaSO_4$).

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Ein solches Verfahren, das im folgenden am Beispiel von Titandioxid beschrieben wird, besteht beispielsweise darin, daß die hydrophobe Oberflächenschicht nach einer Reaktion gemäß

$$n\ TiO_2 + m\ (RO)_3Si\text{-}R' \rightarrow n\ TiO^2\ (\text{oberfl.})$$

erzeugt wird. n und m sind dabei nach Belieben einzusetzende stöchiometrische Parameter, R und R' die gewünschten organischen Reste. Besonders vorteilhaft sind $TiO_2$-Pigmente, beispielsweise die mit Aluminiumstearat beschichteten,

unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Zinkoxide können im Sinne der vorliegenden Erfindung auch in Form kommerziell erhältlicher öliger oder wäßriger Vordispersionen zur Anwendung kommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| ZnO Neutral | / | H&R |
| MZ- 300 | / | Tayca Corporation |
| MZ- 500 | / | Tayca Corporation |
| MZ- 700 | / | Tayca Corporation |
| MZ- 303S | 3% Methicone | Tayca Corporation |
| MZ- 505S | 5% Methicone | Tayca Corporation |
| MZ- 707S | 7% Methicone | Tayca Corporation |
| MZ- 303M | 3% Dimethicone | Tayca Corporation |
| MZ- 505M | 5% Dimethicone | Tayca Corporation |
| MZ- 707M | 7% Dimethicone | Tayca Corporation |
| Z- Sperse Ultra | ZnO (>=56%)/ Ethylhexyl Hydroxystearate Benzoate/ Dimethicone/ Cyclomethicone | Collaborative Laboratories |
| Samt- UFZO- 450/D5 (60%) | ZnO (60%)/ Cyclomethicone/ Dimethicone | Miyoshi Kasei |

Erfindungsgemäße Titandioxid-Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten

Erfindungsgemäße beschichtete und unbeschichtete Titandioxide können in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Geeignete Titandioxidpartikel und Vordispersionen von Titandioxidpartikeln im Sinne der vorliegenden Erfindung sind unter folgenden, Handelsbezeichnungen bei den aufgeführten Firmen erhältlich:

| Handelsname | Coating | zusätzliche Bestandteile der Vordispersion | Hersteller |
|---|---|---|---|
| MT-150W | None | - | Tayca Corporation |
| MT-150A | None | - | Tayca Corporation |
| MT-500B | None | - | Tayca Corporation |
| MT-600B | None | - | Tayca Corporation |
| MT-100TV | Aluminiumhydroxid Stearinsäure | - | Tayca Corporation |
| MT-100Z | Aluminiumhydroxid Stearinsäure | - | Tayca Corporation |
| MT-100T | Aluminiumhydroxid Stearinsäure | - | Tayca Corporation |
| MT-500T | Aluminiumhydroxid Stearinsäure | - | Tayca Corporation |
| MT-100S | Aluminiumhydroxid Laurinsäure | - | Tayca Corporation |
| MT-100F | Stearinsäure Eisenoxid | - | Tayca Corporation |

| Handelsname | Coating | zusätzliche Bestandteile der Vordispersion | Hersteller |
|---|---|---|---|
| MT-100SA | Alumina Silica | - | Tayca Corporation |
| MT-500SA | Alumina Silica | - | Tayca Corporation |
| MT-600SA | Alumina Silica | - | Tayca Corporation |
| MT-100SAS | Alumina Silica Silikon | - | Tayca Corporation |
| MT-500SAS | Alumina Silica Silikon | - | Tayca Corporation |
| MT-500H | Alumina | - | Tayca Corporation |
| MT-100AQ | Silica Aluminiumhydroxid Alginsäure | - | Tayca Corporation |
| Eusolex T | Wasser Simethicone | - | Merck KgaA |
| Eusolex T-2000 | Alumina Simethicone | - | Merck KgaA |
| Eusolex T-Olio F | Silica Dimethylsilate Wasser | $C_{12-15}$ Alkylbenzoate Calcium Polyhydroxystearate Silica Dimethylsilate | Merck KgaA |
| Eusolex T-Olio P | Wasser Simethicone | Octyl Palmitate PEG-7 Hydrogenated Castor Oil Sorbitan Oleate Hydrogenated Castor Oil Beeswax | Merck KgaA |

| Handelsname | Coating | zusätzliche Bestandteile der Vordispersion | Hersteller |
|---|---|---|---|
| | | Stearinsäure | |
| Eusolex T-Aqua | Wasser Alumina Natriummetaphosphat | Phenoxyethanol Natrium Methylparabene Natriummetaphosphat | Merck KgaA |
| Eusolex T-45D | Alumina Simethicone | Isononyl Isononanuate Polyglyceryl Ricinoleate | Merck KgaA |

(fortgesetzt)

| Handelsname | Coating | zusätzliche Bestandteile der Vordispersion | Hersteller |
|---|---|---|---|
| | | Stearinsäure | |
| Kronos 1171 (Titandioxid 171) | None | - | Kronos |
| Titandioxid P25 | None | - | Degussa |
| Titandioxid T805 (Uvinul $TiO_2$) | Octyltrimethylsilan | - | Degussa |
| UV-Titan X610 | Alumina Dimethicone | - | Kemira |
| UV-Titan X170 | Alumina Dimethicone | - | Kemira |
| UV-Titan X161 | Alumina Silica Stearinsäure | - | Kemira |
| UV-Titan M210 | Alumina | - | Kemira |
| UV-Titan M212 | Alumina | Glycerol | Kemira |
| UV-Titan M262 | Alumina Silikon | - | Kemira |
| UV-Titan M160 | Alumina Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Besonders vorteilhaft im Sinne der vorliegenden Erfindung sind Zinkoxide vom Typ Z-Cote HP1 sowie Z-Cote der Fa. BASF und NDM und Neutral von der Firma Haarmann und Reimer (H&R). Desweiteren sind speziell Titandioxide vom Typ Eusolex T-2000 der Fa. Merck, T 805 der Fa. Degussa und MT-100Z und MT-100TV der Fa. Tayca vorteilhaft für den Einsatz in Lichtschutzemulsionen.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid, als auch durch Kombination mehrerer Pigmenttypen innerhalb einer Zubereitung.

Vorzugsweise werden die erfindungsgemäßen Pickering-Emulsionen ferner durch Bornitridpartikel stabilisiert, beispielsweise durch die im folgenden aufgelisteten Bornitride:

| Handelsname | erhältlich bei |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN® | Carborundum |
| Wacker-Bornitrid BNP | Wacker-Chemie |

Vorteilhaft ist es, den mittleren Partikeldurchmesser der verwendeten Bornitridpartikel kleiner als 20 µm, besonders vorteilhaft kleiner als 15 µm zu wählen.

Pickering-Emulsionen im Sinne der vorliegenden Erfindung werden ebenfalls vorteilhaft durch Bornitridpartikel stabilisiert, die oberflächlich wasserabweisend behandelt ("gecoatet") sind, wobei gleichzeitig der amphiphile Charakter gebildet werden bzw. erhalten bleiben soll.

Eine vorteilhafte Beschichtung der Bornitridpartikel besteht aus Dimethylpolysiloxan (Dimethicon). Vorteilhaft sind beispielsweise die bei der Firma Carborundum unter der Handelsbezeichnung Très BN® UHP 1106 erhältlichen, mit Dimethicon behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN® UHP 1107 erhältlichen.

Es ist ferner vorteilhaft, die erfindungsgemäßen Pickering-Emulsionen durch mikrofeine Polymerpartikel zu

stabilisieren.

Vorteilhafte mikrofeine Polymerpartikel sind im Sinne der vorliegenden Erfindung beispielsweise Polycarbonate, Polyether, Polyethylen, Polypropylen, Polyvinylchlorid, Polystyrol, Polyamide, Polyacrylate und dergleichen mehr.

Erfindungsgemäß vorteilhaft sind beispielsweise mikrofeine Polyamid-Partikel, welche unter der Handelsbezeichnung SP-500 bei der Firma TORAY erhältlich sind. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATO-CHEM.

Weitere vorteilhafte Polymerpartikel sind mikrofeine Polymethacrylate. Solche Partikel sind beispielsweise unter der Handelsbezeichnung POLYTRAP® bei der Firma DOW CHEMICAL erhältlich.

Es ist insbesondere vorteilhaft, wenngleich nicht zwingend, wenn die verwendeten mikrofeinen Polymerpartikel oberflächlich beschichtet sind. Diese Oberflächenbehandlung kann darin bestehen, daß die Pigmente nach an sich bekannten Verfahren mit einer dünnen hydrophilen Schicht versehen werden. Vorteilhafte Beschichtungen bestehen beispielsweise aus $TiO_2$, $ZrO_2$ oder auch weiteren Polymeren, wie beispielsweise Polymethylmethacrylat.

Besonders vorteilhafte mikrofeine Polymerpartikel im Sinne der vorliegenden Erfindung sind ferner nach dem in der US-Patentschrift 4,898,913 beschriebenen Verfahren zur hydrophilen Beschichtung hydrophober Polymerpartikel erhältlich.

Vorteilhaft in ist es, den mittleren Partikeldurchmesser der verwendeten mikrofeinen Polymerpartikel kleiner als 100 μm, besonders vorteilhaft kleiner als 50 μm zu wählen. Dabei ist es im wesentlichen unerheblich, in welcher Form (Plättchen, Stäbchen, Kügelchen etc.) die verwendeten Polymerpartikel vorliegen.

Desweiteren ist es vorteilhaft, die erfindungsgemäßen Pickering-Emulsionen durch modifizierte Polysaccharide zu stabilisieren.

Modifizierte Polysaccharide im Sinne der vorliegenden Erfindung sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Solche Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel

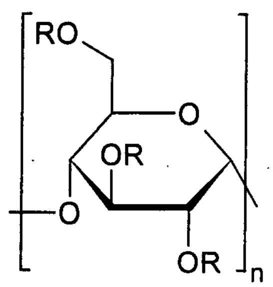

**Strukturformel (I)**

worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte modifizierte Polysaccharide im Sinne der vorliegenden Erfindung.

Besonders vorteilhafte Stärkeether sind z. B. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind ferner Stärkeester und deren Salze, beispielsweise die Natrium- und/oder Aluminiumsalze niedrigsubstituierter Halbester der Stärke, insbesondere Natrium Stärke n-Octenylsuccinat der Strukturformel (I), worin R sich durch die folgende Struktur auszeichnet

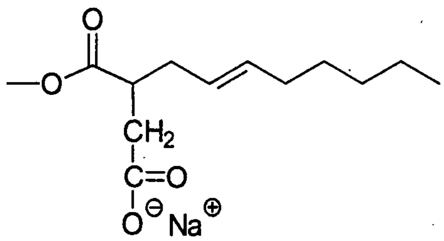

und welches z. B. unter der Handelsbezeichnung Amiogum® 23 bei der Firma CERESTAR erhältlich ist sowie Aluminium Stärke Octenylsuccinate, insbesondere die unter den Handelsbezeichnungen Dry Flo® Elite LL und Dry Flo® PC bei der Firma CERESTAR erhältlichen.

Vorteilhaft ist es, den mittleren Partikeldurchmesser der verwendeten modifizierten Polysaccharide kleiner als 20 µm, besonders vorteilhaft kleiner als 15 µm zu wählen.

Die Liste der genannten modifizierten Polysaccharide, die erfindungsgemäße Pickering-Emulsionen stabilisieren können, soll selbstverständlich nicht limitierend sein. Modifizierte Polysaccharide im Sinne der vorliegenden Erfindung sind auf zahlreichen, an sich bekannten Wegen, sowohl chemischer als auch physikalischer Natur erhältlich.

Die vorstehend genannten amphiphilen Partikel eignen sich hervorragend sowohl zur Stabilisierung von W/O-Pickering-Emulsionen als auch zur Stabilisierung von O/W-Pikkering-Emulsionen. Nachfolgend werden erfindungsgemäße mikrofeine Partikel genannt, welche vorteilhaft insbesondere einen der beiden Emulsionstypen W/O bzw. O/W stabilisieren.

Die erfindungsgemäßen kosmetischen und dermatologischen Zubereitungen können Farbstoffe und/oder Farbpigmente enthalten, insbesondere wenn sie in Form von dekorativen Lippenstiften, Lippenkonturenstiften, Abdeckstiften, Kajalstiften, Augenkonturenstiften und/oder Augenbrauenstiften vorliegen. Die Farbstoffe und - pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. (Die Stoffe sind nach ihrer Colour Index Number geordnet.)

| Colour Index Number | Deutsche Bezeichnung | CAS-Nr. bzw. Summenformel | Kategorie |
|---|---|---|---|
| 10316 | C-ext. Gelb 1 | 846-70-8 | B |
| 12075 | C-Orange | 3468-63-1 | A |
| 14700 | C-Rot 57 | 4548-53-2 | B |
| 15510 | C-ext.Orange 8 | 633-96-5 | B |
| 15585 | C-Rot 55 | 2092-56-0 | B |
| 15585:1 | C-Rot 55 | 5160-02-1 | B |
| 15800:1 | C-ext. Rot 57 | 6371-76-2 | A |
| 15850 | Lithol Rubin 8 | 5858-81-1 | A |
| 15850:1 | C-Rot 12 | 5281-04-9 | A |
| 15880:1 | C-ext. Rot 61 | 6417-83-0 | A |
| 15980 | C-Orange 9 | $C_{16}H_{10}N_2O_7S_2 \cdot 2Na$ | A |
| 15985 | C-Orange 10 | 2783-94-0 | A |
| 16035 | C-Rot 60 | 29956-17-6 | A |
| 17200 | C-Rot 58 | $C_{16}H_{13}N_3O_7S_2$ 2Na | A |
| 19140 | C-Gelb 10 | 1934-21-0 | A |

| Colour Index Number | Deutsche Bezeichnung | CAS-Nr. bzw. Summenformel | Kategorie |
|---|---|---|---|
| 20170 | C-ext. Braun 4 | 1320-07-6<br>6371-84-2 | A |
| 26100 | C-ext. Rot 56 | 85-86-9 | A |

(fortgesetzt)

| Colour Index Number | Deutsche Bezeichnung | CAS-Nr. bzw. Summenformel | Kategorie |
|---|---|---|---|
| 42053 | C-Grün 12 | $C_{37}H_{36}N_2O_{10}S_3$ 2Na | A |
| 42090 | C-Blau 21 | 2650-18-2 | A |
| 42090 | C-Blau 21 (Ammonium Salz) | 2650-18-2<br>6371-85-3<br>37307-56-5 | A |
| 45170 | C-Rot 59 | 81-88-9 | B |
| 45170:1 | (Rhodamin B-stearat) | $C_{28}H_{31}N_2O_3 \cdot C_{18}H_{35}O_2$ | B |
| 45370:1 | C-Rot 27 | $C_{20}H_{10}Br_2O_5$ | A |
| 45380 | C-Rot 30 | 17372-87-1 | A |
| 45380:2 | Tetrabromfluoreszein | 15086-94-9 | A |
| 45410 | C-Rot 34 | 18472-87-2 | A |
| 45410:1 | Tetrabromtetrachlorfluoreszein | 13473-26-2 | A |
| 45425 | C-Rot 35 | $C_{20}H_{10}I_2O_5$ 2Na | A |
| 45425:1 | Fluoreszein-Gemisch | 518-40-7<br>38577-97-8 | A |
| 47000 | C-ext. Gelb 23 | 8003-22-3 | B |
| 47005 | C-Gelb 11 | 8004-92-0 | A |
| 59040 | C-ext. Gelb 24 | 6358-69-6 | A |
| 60725 | C-ext. Violett 18 | 81-48-1 | A |
| 61565 | C-Grün 10 | 128-80-3 | A |
| 61570 | C-Grün 11 | 4403-90-1 | A |
| 73360 | C-Rot 28 | 2379-74-0 | A |
| 75120 | C-Orange 12 | 8015-67-6 | A |
| 75130 | C-Orange 11 | 7235-40-7 | A |
| 75170 | Guanin | 68-94-0<br>73-40-50 | A |
| 75470 | C-Rot 50 | $C_{22}H_{20}O_{13}$ | A |

| Colour Index Number | Deutsche Bezeichnung | CAS-Nr. bzw. Summenformel | Kategorie |
|---|---|---|---|
| 75480 | Henna | $C_{10}H_6O_3$ (Lawson) | A |
| 75810 | C-Grün 8 | 11006-34-1 | A |
| 75810 | C-Grün 7 | 479-61-8 | A |
| | | 519-62-0 | A |
| 77000 | C-Pigment 1 | Al | A |
| 77007 | C-Blau 16 | 57455-37-5 | A |
| 77019 | C-Weiß 11 | 12001-26-2 | A |
| 77288 | C-Grün 9 | 1308-38-9 | A |
| 77289 | C-Grün 14 | 12001-99-9 | A |
| 77400 | Bronze | 7440-50-8 | A |
| 77491 | C-Rot 45 | 1309-37-1 | A |
| 77492 | C-Braun 3 (C-Gelb 8) | $Fe_2O_3FeO(OH)$ | A |
| 77499 | C-Schwarz 5 | $Fe_3O_4$ | A |
| 77510/20 | C-Blau 17 | $C_6FeN_6 \cdot 4/3$ Fe | A |
| 77742 | C-Violett 11 | 10101-66-3 | A |
| 77820 | C-Pigment 2 | 7440-22-4 | A |
| 77891 | C-Weiß 7 | 13463-67-7 ($TiO_2$) | A |
| 77947 | C-Weiß 8 | 1314-13-2 | A |

Die Kategorien (A) und (B) sind folgendermaßen definiert:

**(A)**

Der Farbstoff kann verwendet werden für die Herstellung von oral zu verabreichenden Zubereitungen, wie z. B. Lippenpflegemittel, für Zubereitungen, die im Bereich der Augen zur Anwendung kommen, für externe Zubereitungen (Cremes, Lotionen, Puder), für Zubereitungen, welche wieder abgespült werden (Rinse-off [RO]-Products, wie z. B. Haarwaschmittel, Haarkonditioniermittel usw.), für Haarfärbemittel, für Nagelpflegemittel.
Eine Kennzeichnung der Farbstoffe ist nicht erforderlich.

**(B)**

Der Farbstoff kann verwendet werden für die Herstellung von Zubereitungen, die unter (A) gelistet werden, jedoch nicht für die Herstellung von Präparaten, die im Bereich der Augen zur Anwendung kommen.
Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden.
Die Liste der genannten Farbstoffe und Farbpigmente, die in den erfindungsgemäßen Pickering-Emulsionsstiften verwendet werden können, soll selbstverständlich nicht limitierend sein.
Erfindungsgemäße Pickering-Emulsionen können vorteilhaft auch Verdickungsmittel, insbesondere auch Ölverdickungsmittel enthalten, um die taktilen Eigenschaften der Emulsion und die Stiftkonsistenz zu verbessern. Vorteilhafte Ölverdickungsmittel im Sinne der vorliegenden Erfindung sind beispielsweise weitere Feststoffe, wie z. B. hydrophobe Siliciumoxide des Typs Aerosil®, welche von der Degussa AG erhältlich sind. Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974 und/oder Aerosil® R976.
Weitere vorteilhafte Ölverdickungsmittel modifizierte Schichtsilikate.
Silikate sind Salze und Ester (Kieselsäureester) der Orthokieselsäure [$Si(OH)_4$] und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten. Schichtsilikate (Phyllosilikate, Blattsilikate) sind (idealerweise) Silikat-Strukturen mit zweidimensional unendlichen Schichten aus $[SiO_4]^{4-}$-Tetraedern, wobei jedes Tetraeder über 3 Brücken-Sauerstoffe mit Nachbar-Tetraedern verbunden ist.
Chemische Formeln lassen sich für Schichtsilikate nur angenähert aufstellen, da sie ein großes Ionenaustausch-Vermögen besitzen und Silizium gegen Aluminium und dieses wiederum gegen Magnesium, $Fe^{2+}$, $Fe^{3+}$, Zn und dergleichen ausgetauscht werden kann. Die daraus möglicherweise resultierende negative Ladung der Schichten wird in der Regel durch Kationen, insbesondere durch $Na^+$ und $Ca^{2+}$ in Zwischenschicht-Positionen ausgeglichen.
Schichtsilikate können durch reversible Einlagerung von Wasser (in der 2- bis 7-fachen Menge) und anderen Substanzen wie z. B. Alkoholen, Glykolen und dergleichen mehr aufquellen. Ihre Verwendung als Verdickungsmittel in kosmetischen Mitteln ist dementsprechend an sich bekannt. Allerdings konnte der Stand der Technik nicht den Weg zur vorliegenden Erfindung weisen.
Vorteilhafte Schichtsilikate, welche in einer erfindungsgemäßen Kombination mit Bornitrid eingesetzt werden können, sind beispielsweise solche, deren größte Ausdehnungsrichtung im unmodifizierten und ungequollenen Zustand im Mittel eine Länge von weniger als 10 µm hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Substanzen ab.
Vorteilhafte modifizierte Schichtsilikate im Sinne der vorliegenden Erfindung sind beispielsweise modifizierte Smektite (Smectite).
Smektite sind stets sehr feinkörnige (meist < 2 mm), überwiegend als lamellenförmige, moosartige oder kugelförmige Aggregate vorkommende Dreischicht-Tonminerale (2:1-Schichtsilikate), in denen eine zentrale Schicht aus oktaedrisch koordinierten Kationen sandwichartig von 2 Schichten aus $[(Si,Al)O_4]$-Tetraedern umgeben ist. Smektite werden idealisiert durch die folgende Strukturformel beschrieben, worin weiß ausgefüllte Kreise Silizium- und/oder Aluminiumatome, hellgrau ausgefüllte Kreise Sauerstoffatome, dunkelgrau ausgefüllte Kreise Wasserstoffatome und schwarz ausgefüllte Kreise Aluminium-, Magnesium-, Eisenatome und/oder weitere Austauschkationen darstellen:

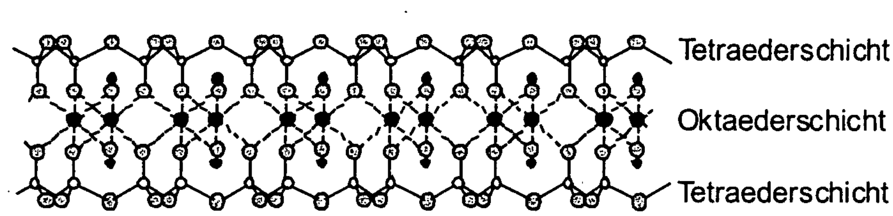


Vorteilhafte modifizierte Smektite sind z. B. modifizierte Montmorillonite. Montmorillonite werden durch die angenäherte chemische Formel $Al_2[(OH)_2/Si_4O_{10}]\cdot$ n $H_2O$ bzw. $Al_2O_3 \cdot 4\ SiO_2 \cdot H_2O \cdot$ n $H_2O$ beschrieben und stellen zu den dioktaedrischen Smektiten gehörende Tonmineralien dar.

Besonders vorteilhaft im Sinne der vorliegenden Erfindung sind ferner beispielsweise modifizierte Hektorite. Hektorite gehören zu den Smektiten und haben die angenäherte chemische Formel $M^+_{0,3}(Mg_{2,7}Li_{0,3)}[Si_4O_{10}(OH)_2]$, worin $M^+$ meist $Na^+$ darstellt.

Vorteilhaft im Sinne der vorliegenden Erfindung sind ferner modifizierte Bentonite. Bentonite sind Tone und Gesteine, die Smektite, vor allem Montmorillonit, als Hauptminerale enthalten. Die "Roh"-Bentonite sind entweder Calcium-Bentonite (in Großbritannien als Fuller-Erden bezeichnet) oder Natrium-Bentonite (auch: Wyoming-Bentonite).

Modifizierte Schichtsilikate im Sinne der vorliegenden Erfindung sind Schichtsilikate, insbesondere die bereits genannten Schichtsilikattypen, deren Organophilie (auch: Lipophilie) - beispielsweise durch Umsetzung mit quarternären Ammonium-Verbindungen - erhöht wurde. Solche Schichtsilikate werden auch als organophile Schichtsilikate bezeichnet.

Besonders vorteilhaft im Sinne der vorliegenden Erfindung sind sogenannte Bentone, d. h. organische Derivate von Montmorilloniten (bzw. Bentoniten) und/oder Hectoriten, die durch Ionenaustausch-Reaktionen mit Alkylammonium-Basen hergestellt werden.

Vorteilhafte modifizierte Schichtsilikate im Sinne der vorliegenden Erfindung sind beispielsweise durch Umsetzung von Schichtsilikaten mit Quatemium-18 erhältlich. Quaternium-18 ist eine Mischung von quaternären Ammoniumchloridsalzen, welche durch die folgende Strukturformel beschrieben werden:

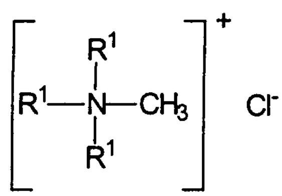

worin
die Reste $R^1$ unabhängig voneinander gewählt werden aus der Gruppe Methyl und hydrierte Talgreste mit einer Kettenlänge von 12 bis 20 Kohlenstoffatomen.

Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18, welche z. B. unter den Handelsbezeichnungen Bentone 27 und Bentone 38 bei Nordmann & Rassmann erhältlich sind.

Die Gesamtmenge an einem oder mehreren modifizierten Schichtsilikaten in den fertigen kosmetischen oder dermatologischen Zubereitungen wird vorteilhaft aus dem Bereich von 0,05 bis 5,0 Gew.-%, bevorzugt 0,1 bis 1,0 Gew.-% gewählt, bezogen auf das Gesamtgewicht der Zubereitungen.

Ferner sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder Magnesium-Stearat.

W/O-Pickering-Emulsionen:

Der Wasserphasenanteil der erfindungsgemäßen W/O-Pickering-Emulsionen wird vorzugsweise aus dem Bereich von 0,5 bis 75 Gew. % gewählt, bezogen auf das Gesamtgewicht der Formulierungen.

Vorteilhaft zur Stabilisierung von W/O-Pickering-Emulsionen sind insbesondere auch Magnesiumsilicate (auch: Talkum), beispielsweise die unter der Handelsbezeichnung Talkum Micron bei der Firma Grolmann erhältlichen.

O/W-Pickering-Emulsionen:

Der Fettphasenanteil der erfindungsgemäßen O/W-Pickering-Emulsionen wird vorzugsweise aus dem Bereich von 0,5 bis 75 Gew. % gewählt, bezogen auf das Gesamtgewicht der Formulierungen.

Besonders vorteilhaft zur Stabilisierung von O/W-Pickering-Emulsionen sind im Sinne der vorliegenden Erfindung auch unbehandelte, nahezu reine Pigmentpartikel, beispielsweise Titandioxid-Pigmente, insbesondere solche, die unter der Handelsbezeichnung KRO-NOS® 1171 ($TiO_2$) von der Firma Kronos Titan erhältlich sind.

O/W-Pickering-Emulsionen werden im Sinne der vorliegenden Erfindung ferner besonders vorteilhaft durch Metalloxidpartikel stabilisiert, die mit Aluminiumhydroxid und/oder Siliziumdioxid überzogen ("gecoatet") sind. Vorteilhafte Ausführungsformen sind beispielsweise Titandioxidpartikel, die unter dem Namen EUSOLEX® TA bei der Firma Merck erhältlich sind.

Es ist ferner vorteilhaft, wenngleich nicht zwingend, die erfindungsgemäßen mikrofeinen Partikel mit weiteren amphiphilen Partikeln zu kombinieren, welche gegebenenfalls auch zur Stabilisierung der Pickering-Emulsionen beitragen können.

Solche Partikel sind beispielsweise Titandioxidpigmente, die mit Octylsilanol beschichtet sind, und/oder Siliciumdioxidpartikel, die oberflächlich wasserabweisend behandelt sind. Geeignete Siliciumdioxidpartikel sind beispielsweise sphärische Polyalkylsilsesquioxan-Partikel, wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich. Geeignete Titandioxidpartikel sind unter der Handelsbezeichnung T805 ebenfalls bei der Firma Degussa erhältlich.

Die erfindungsgemäßen Pickering-Emulsionen können als Grundlage für kosmetische oder dermatologische Formulierungen dienen. Diese können wie üblich zusammengesetzt sein und beispielsweise zur Behandlung und der Pflege der Haut, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika üblichen Weise auf die Haut in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tagesoder Nachtcrème usw. Es ist gegebenenfalls möglich und vorteilhaft, die erfindungsgemäßen Zusammensetzungen als Grundlage für pharmazeutische Formulierungen zu verwenden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Erfindungsgemäße Pickering-Emulsionen können auch Verdickungsmittel enthalten, um die taktilen Eigenschaften der Emulsion zu verbessern.

Insbesondere können die erfindungsgemäßen Pickering-Emulsionen auch Antioxidantien enthalten. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole(z.B. Urocaninsäure) und deren Derivate, Peptide wie D, L-Carnosin, D-Carnosin, L-Carnosin und . deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon,

Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Günstig sind auch kosmetische und dermatologische Zubereitungen, die in der Form eines Sonnenschutzmittels vorliegen. Vorzugsweise enthalten diese mindestens eine weitere UV-A-Filtersubstanz und/oder mindestens eine UV-B-Filtersubstanz und/oder mindestens ein weiteres anorganisches Pigment aus der Gruppe der Oxide des Eisens, Zirkoniums, Siliciums, Mangans, Aluminiums, Cers und Mischungen davon sowie Abwandlungen, bei denen die Oxide die aktiven Agentien sind.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen Substanzen enthalten, die UV-Strahlung im UV-B-Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Enthalten die erfindungsgemäßen Emulsionen UV-B-Filtersubstanzen, können diese öllöslich oder wasserlöslich sein. Erfindungsgemäß vorteilhafte öllösliche UV-B-Filter sind z.B.:

- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- hinsichtlich der $C_3$-Achse des Triazingrundkörpers symmetrisch Triazinderivate,
- Benzotriazolderivate, vorzugsweise 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist
- sowie an Polymere gebundene UV-Filter,
- 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Vorteilhafte wasserlösliche UV-B-Filter sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Triazinderivate sind z.B.:

- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist;
- Diethylhexylbutylamidotriazon (INCI: Diethylhexylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches unter der Handelsbezeichnung Mexoryl® XL bei der Fa. Chimex erhältlich ist.

Die weiteren UV-Filtersubstanzen können öllöslich oder wasserlöslich sein.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethylhexyloxy)-2-hydroxy]-phenyl}-6-(4-methoxyphenyl)-1,3,5-triazin, jeweils einzeln oder in beliebigen Kombinationen miteinander.

Die Liste der genannten UV-B-Filter, die in den erfindungsgemäßen Pickering-Emulsionen verwendet werden können, soll selbstverständlich nicht limitierend sein.

Es kann auch von Vorteil sein, in erfindungsgemäßen Pickering-Emulsionen UV-A-Filter zu verwenden, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:

- Phenylen-1,4-bis-(2-benzimidazyl)-3,3'-5,5'-tetrasulfonsäure

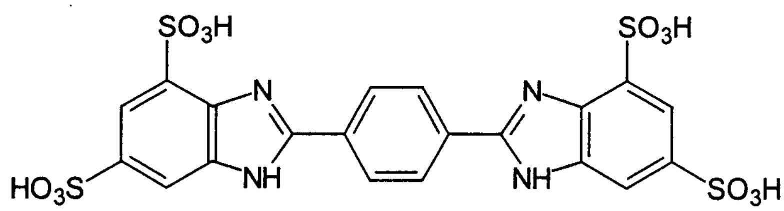

und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz

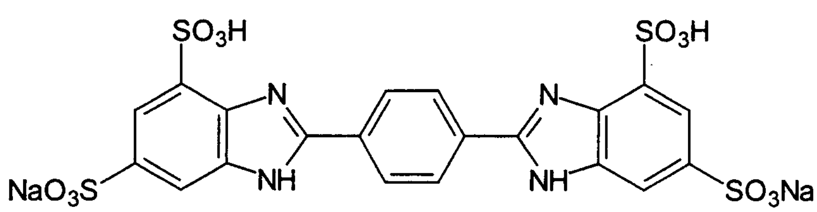

mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;

- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfatoverbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

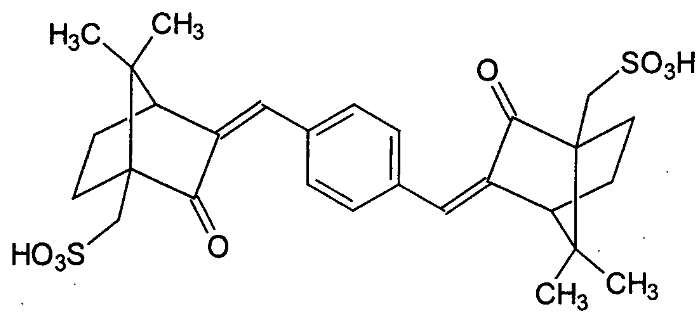

Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;

Auch Zubereitungen, die UV-A-Filter enthalten, sind Gegenstand der Erfindung. Es können die für die UV-B-Kombination verwendeten Mengen eingesetzt werden.

Vorteilhaft können erfindungsgemäße Zubereitungen außerdem als Grundlage für kosmetische Desodorantien bzw. Antitranspirantien eingesetzt werden, so daß die vorliegende Erfindung in einer besonderen Ausführungsform Pickering-Emulsionen als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen W/O-Emulsionsstifte eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Liste der genannten Wirkstoffe bzw. Wirkstoffkombinationen soll selbstverständlich nicht limitierend sein.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Rezepturbeispiele:**

| | W/O Pick Cr. 1 | W/O Pick Cr. 2 | W/O Pick Cr. 3 |
|---|---|---|---|
| Octyldodecanol | 5,50 | 5,00 | 5,00 |
| C12-15- Alkyl Benzoat | 6,50 | 6,00 | |
| Butylen Glycol Caprylate/Caprat | | 4,50 | 4,50 |
| Dicaprylyl Ether | 5,50 | 3,50 | 2,50 |
| Dicaprylyl Carbonat | | | 6,00 |
| Hydroxyoctacosanyl Hydroxystearat | 2,00 | 2,50 | 2,00 |
| Disteardimonium Hectorit | 1,00 | 0,50 | 1,00 |
| Dimethicon | | | 2,50 |
| Aminobenzophenon | 2,00 | 1,00 | 0,50 |
| Butyl Methoxydibenzoylmethan | 0,50 | 1,00 | 1,50 |
| Methylbenzylidene Campher | | 2,00 | |
| Ethylhexyltriazon | | | 2,00 |
| Bis-Ethylhexyloxyphenol Methoxyphenyl Triazin | | | 1,70 |
| Diethylhexyl Butamido Triazon | | | 0,5 |
| Methylene Bis-Benzotriazolyl Tetramethylbutylphenol | 2,00 | | |
| Titandioxid + Alumina + Simethicon + Aqua | 0,50 | 0,50 | 0,50 |
| Titandioxid + Trimethoxycaprylylsilan | 3,50 | 2,50 | |
| Zinkoxid Dimethicon | | 2,00 | |
| Bariumsulfat | 0,50 | | |
| Bornitrid | 2,00 | 1,50 | |
| Distärke Phosphat | | | 1,50 |
| 2,6 Diethylhexyl-naphthalat | | 4,00 | |
| Natrium Chlorid | 1,00 | | 1,00 |
| Magnesium Sulfat | | 0,70 | |
| Glycerin | 10,00 | 5,00 | 10,00 |
| Trisodium EDTA | 1,00 | 1,00 | 1,00 |
| Porpylene Carbonat | 0,33 | | 0,33 |
| Methylparaben | 0,20 | 0,30 | |
| Propylparaben | | 0,07 | |
| Phenoxyethanol | 0,50 | 0,50 | 0,40 |
| Hexamidine Diisethionat | 0,08 | 0,08 | |
| Diazolidinyl Urea | | | 0,28 |
| Parfüm | 0,40 | 0,40 | 0,20 |
| Wasser | Ad 100 | Ad 100 | Ad 100 |

| | W/O Pick-Cr. 4 | WO Pick-Cr. 5 | WO Pick-Cr. 6 |
|---|---|---|---|
| Mineralöl | 6,00 | | 10,00 |
| Octyldodecanol | 5,00 | | |
| Caprylic/Capric Triglycerid | 6,00 | | |
| C12-15- Alkyl Benzoat | | | 5,00 |
| Dicaprylyl Ether | | | 4,00 |
| Hydroxyoctacosanyl Hydroxystearat | 1,00 | 1,50 | 2,50 |
| Disteardimonium Hectorit | | 1,50 | |
| Dimethicon | | 9,00 | |
| Shea Butter | | | 0,50 |
| Vitamin E-Acetat | 0,50 | 1,00 | 1,00 |
| Aminobenzophenon | 1,50 | 1,00 | 2,00 |
| Ethylhexylmethoxycinnamat | | 5,00 | |
| Ethylhexylsalicylat | | 2,50 | |
| Octocrylen | | 2,50 | |
| Isopropyl Dibenzoyl Methan | | | 1,00 |
| Butyl Methoxydibenzoylmethan | 0,50 | 1,50 | |
| Ethylhexyltriazon | 1,50 | | |
| Drometritriazol Trisiloxan | | | 1,00 |
| Titandioxid + Alumina + Simethicon + Aqua | 3,00 | 4,00 | 1,50 |
| Titandioxid + Trimethoxycaprylylsilan | | | 1,50 |
| Terephthalidene Dicampher Sulfonsäure | | | 0,50 |
| Phenylbenzimidazol Sulfonsäure | 1,00 | | |
| Silica Dimethyl Silylat | 1,00 | 0,50 | 0,50 |
| Natrium Chlorid | 1,00 | | 1,00 |
| Magnesium Sulfat | | 0,70 | |
| Glycin | | 1,00 | |
| Citronensäure | | | 0,086 |
| Natrium Citrat | | | 0,174 |
| Glycerin | 3,00 | 10,00 | |
| Butyleneglycol | | | 7,50 |
| Trisodium EDTA | | 0,75 | 0,50 |
| Methylparaben | | | 0,20 |
| Phenoxyethanol | 0,40 | 0,50 | 0,50 |
| Hexamidine Diisethionat | | | 0,08 |
| Diazolidinyl Urea | 0,28 | | |
| DMDM Hydantoin | | 0,60 | |
| Etahnol | 3,00 | | |
| Parfüm | 0,50 | 0,50 | 0,40 |

| | | | |
|---|---|---|---|
| Wasser | Ad 100 | | Ad 100 |

| | W/O Pick-Cr. 7 |
|---|---|
| C12-15- Alkyl Benzoat | 8,00 |
| Butylen Glycol Caprylat/Caprat | 8,00 |
| Hydroxyoctacosanyl Hydroxystearat | 1,50 |
| Disteardimonium Hectorit | 0,25 |
| Cera Microcristallina + Paraffinum Liquidum | 5,00 |
| Verdicker (Hydroxypropyl Methylcellulose) | 0,05 |
| Dimethicon | 3,0 |
| Aminobenzophenon | 1,00 |
| Isopropyl Dibenzoyl Methan | 0,50 |
| Butyl Methoxydibenzoylmethan | 0,50 |
| Methylbenzylidene Campher | 2,00 |
| Ethylhexyltriazon | 2,00 |
| Titandioxid + Alumina + Simethicon + Aqua | 1,50 |
| Titandioxid + Trimethoxycaprylylsilan | 3,00 |
| Zinkoxid Dimethicon | 2,00 |
| Natrium Chlorid | 1,00 |
| Glycerin | 10,00 |
| Methylparaben | 0,20 |
| Phenoxyethanol | 0,50 |
| Hexamidine Diisethionat | 0,08 |
| Parfüm | 0,50 |
| Wasser | Ad 100 |

| | O/W Pick-Cr. 8 |
|---|---|
| Octyldodecanol | 3,00 |
| Caprylic/Capric Triglycerid | 3,00 |
| C12-15- Alkyl Benzoat | 3,00 |
| PVP/Hexadecen Copolymer | 0,50 |
| Sodium Carbomer | 0,10 |
| Xanthan Gum | 0,10 |
| Glyceryl Stearat Citrat | 0,60 |
| Cyclomethicon | 2,00 |
| Vitamin E-Acetat | 0,50 |
| Aminobenzophenon | 0,50 |
| Ethylhexylmethoxycinnamat | 7,50 |
| Butyl Methoxydibenzoylmethan | 2,00 |
| Bis-Ethylhexyloxyphenol Methoxyphenyl Triazin<br>Titandioxid + Alumina + Simethicon + Aqua | 1,00<br>0,60 |
| Phenyl Dibenzimidazol Tetrasulfonsäure | 0,5% |
| Distärke Phosphat | 6,00 |
| Silica | 0,60 |
| Citronensäure | 0,086 |
| Sodium Citrat | 0,174 |
| Glycerin | 4,00 |
| Butyleneglycol | 4,00 |
| Octoxyglycerin | 0,30 |
| Trisodium EDTA | 1,00 |
| Glucosylrutin<br>Methylparaben | 0,50<br>0,20 |
| Phenoxyethanol | 0,30 |
| Iodopropynyl Butylcarbamat | 0,18 |
| Etahnol | 5,00 |
| Parfüm | 0,30 |
| Wasser | Ad 100 |

| | O/W-Pick-Cr. 9 |
|---|---|
| Octyldodecanol | 19,00 |
| C12-15- Alkyl Benzoat | 19,00 |
| Dicaprylyl Ether | 8,00 |
| Hydrogenated Polyisobuten | 7,00 |
| Disteardimonium Hectorit | 0,50 |
| Xanthan Gum | 0,20 |
| Phenyltrimethicon | 2,00 |
| Vitamin E-Acetat | 1,00 |
| Aminobenzophenon | 3,00 |
| Octocrylen | 5,00 |
| Isopropyl Dibenzoyl Methan | 0,50 |
| Titandioxid + Alumina + Simethicon + Aqua | 1,50 |
| Corn Stärke Modified | 1,00 |
| Glycerin | 7,50 |
| Trisodium EDTA | 0,50 |
| Ubiquinon | 0,25 |
| Phenoxyethanol | 0,50 |
| Diazolidinyl Urea | 0,28 |
| Wasser | Ad 100 |

| | W/O Lotin 10 | Pick-Lotion 11 |
|---|---|---|
| Mineralöl | | 16,00 |
| Octyldodecanol | | 5,00 |
| Caprylic/Capric Triglycerid | | 6,00 |
| C12-15- Alkyl Benzoat | 7,50 | |
| Butylen Glycol Caprylat/Caprat | 7,50 | |
| Dicaprylyl Ether | 7.50 | |
| Hydroxyoctacosanyl Hydroxystearat | 2,00 | 1,50 |
| PVP/Hexadecen Copolymer | 0,75 | 1,00 |
| Disteardimonium Hectorit | 0,50 | 1,00 |
| Cetyl PEG/PPG-10/1 Dimethicon | 0,50 | |
| Shea Butter | 0,50 | |
| Phenyltrimethicon | | |
| Cyclomethicon | 2,00 | 2,00 |
| Vitamin E-Acetat | 0,50 | |
| Aminobenzophenon | 1,00 | 2,00 |
| Ethylhexylmethoxycinnamat | 3,00 | |
| Butyl Methoxydibenzoylmethan | 1,50 | 0,50 |
| Ethylhexyltriazon | | 1,50 |
| Diethylhexyl Butamido Triazon | 2,00 | |
| Titandioxid + Alumina + Simethicon + Aqua | 0,50 | 2,00 |
| Zinkoxid Dimethicon | | 2,00 |
| Phenylbenzimidazole Sulfonsäure | 0,50 | |
| Silica Dimethyl Silylate | | 0,50 |
| Bornitrid | 2,00 | |
| Magnesium Sulfat | | 0,70 |
| Zink Sulfat | 1,40 | |
| Glycin | 1,50 | |
| Glycerin | 10,00 | 3,00 |
| Butyleneglycol | | 5,00 |
| Trisodium EDTA | 1,00 | 1,00 |
| Porpylen Carbonat | | 0,33 |
| Glucosylrutin<br>Methylparaben | 0,50 | <br>0,21 |
| Propylparaben | | 0,07 |
| Phenoxyethanol | 0,40 | 0,50 |
| DMDM Hydantoin | 0,60 | |
| Etahnol | 2,00 | |
| Parfüm | 0,40 | |
| Wasser | Ad 100 | Ad 100 |

| | W/O Pick Cr. 12 | W/O Pick Cr. 13 |
|---|---|---|
| Octyldodecanol | 5,50 | 5,00 |
| C12-15- Alkyl Benzoat | 6,50 | 6,00 |
| Butylen Glycol Caprylat/Caprat | | 4,50 |
| Dicaprylyl Ether | 5,50 | 3,50 |
| Hydroxyoctacosanyl Hydroxystearat | 2,00 | 2,50 |
| Disteardimonium Hectorit | 1,00 | 0,50 |
| Aminobenzophenon | 2,00 | 1,00 |
| Butyl Methoxydibenzoylmethan | | 1,00 |
| Methylbenzylidene Campher | | 2,00 |
| Ethylhexyltriazon | | 1,00 |
| Methylene Bis-Benzotriazolyl Tetramethylbutylphenol | 2,00 | |
| Titandioxid + Alumina + Simethicon + Aqua | | 0,50 |
| Titandioxid + Trimethoxycaprylylsilan | 4,00 | 2,00 |
| Zinkoxid | 2,00 | |
| Bariumsulfat | 0,50 | |
| Bornitrid | 2,00 | 1,50 |
| Natrium Chlorid | 1,00 | |
| Magnesium Sulfat | | 0,70 |
| DHA | | 5,00 |
| Glycerin | 10,00 | 5,00 |
| Trisodium EDTA | 1,00 | 1,00 |
| Porpylene Carbonat | 0,33 | |
| Repellent 3535 | | 2,00 |
| Methylparaben | 0,20 | 0,30 |
| Propylparaben | | 0,07 |
| Phenoxyethanol | 0,50 | 0,50 |
| Hexamidine Diisethionat | 0,08 | 0,08 |
| Parfüm | 0,40 | 0,40 |
| Wasser | Ad 100 | Ad 100 |

| | W/O Pick Cr. 14 |
|---|---|
| Octyldodecanol | 5,00 |
| Butylen Glycol Caprylat/Caprat | 4,50 |
| Dicaprylyl Ether | 2,50 |
| Dicaprylyl Carbonat | 6,00 |
| Hydroxyoctacosanyl Hydroxystearat | 2,00 |
| Disteardimonium Hectorit | 1,00 |
| Dimethicon | 2,50 |
| Aminobenzophenon | 0,50 |
| Ethylhexylmethoxycinnamat | 5,00 |
| Ethylhexyltriazon | 1,50 |
| Bis-Ethylhexyloxyphenol Methoxyphenyl Triazin | 1,70 |
| Titandioxid + Alumina + Simethicon + Aqua | 1,00 |
| Distärke Phospaht | 1,50 |
| Natrium Chlorid | 1,00 |
| Glycerin | 10,00 |
| Trisodium EDTA | 1,00 |
| Porpylene Carbonat | 0,33 |
| Phenoxyethanol | 0,40 |
| Diazolidinyl Urea | 0,28 |
| Parfüm | 0,20 |
| Wasser | Ad 100 |

| | W/O Pick 15 | WO Pick-Cr. 16 |
|---|---|---|
| Mineralöl | 6,00 | |
| Octyldodecanol | 5,00 | |
| Caprylic/Capric Triglycerid | 6,00 | |
| Hydroxyoctacosanyl Hydroxystearat | 1,00 | 1,50 |
| Disteardimonium Hectorit | | 1,50 |
| Dimethicon | | 9,00 |
| Vitamin E-Acetat | 0,50 | 1,00 |
| Aminobenzophenon | 3,00 | 0,50 |
| Ethylhexylmethoxycinnamat | 5,00 | 7,50 |
| Ethylhexylsalicylat | | 5,00 |
| Octocrylen | 5,00 | |
| Butyl Methoxydibenzoylmethan | | 1,50 |
| Drometritriazole Trisiloxan | 0,50 | |
| Titandioxid + Alumina + Simethicon + Aqua<br>Silica Dimethyl Silylat | 3,00<br>1,00 | 4,00<br>0,50 |
| Natrium Chlorid | 1,00 | |
| Magnesium Sulfat | | 0,70 |
| Glycin | | 1,00 |
| Glycerin | 3,00 | 10,00 |
| Trisodium EDTA | | 0,75 |
| Phenoxyethanol | 0,40 | 0,50 |
| Diazolidinyl Urea | 0,28 | |
| DMDM Hydantoin | | 0,60 |
| Etahnol | 3,00 | |
| Parfüm | 0,50 | 0,50 |
| Wasser | Ad 100 | Ad 100 |

| | WO Pick-Cr. 17 | W/O Pick-Cr. 18 |
|---|---|---|
| Mineralöl | 10,00 | |
| C12-15- Alkyl Benzoat | 5,00 | 8,00 |
| Butylen Glycol Caprylat/Caprat | | 8,00 |
| Dicaprylyl Ether | 4,00 | |
| Hydroxyoctacosanyl Hydroxystearat | 2,50 | 1,50 |
| Disteardimonium Hectorit | | 0,25 |
| Cera Microcristallina + Paraffinum Liquidum | | 5,00 |
| Verdicker (Hydroxypropyl Methylcellulose) | | 0,05 |
| Dimethicon | | 3,0 |
| Shea Butter | 0,50 | |
| Vitamin E-Acetat | 1,00 | |
| Aminobenzophenon | 2,00 | 1,50 |
| Ethylhexylmethoxycinnamat | 2,50 | |
| Octocrylen | 2,50 | |
| Bis-Ethylhexyloxyphenol Methoxyphenyl Triazin | | 2,00 |
| Methylen Bis-Benzotriazolyl Tetramethylbutylphenol | | 4,00 |
| Titandioxid + Alumina + Simethicone + Aqua | 2,00 | 1,50 |
| Titandioxid + Trimethoxycaprylylsilan | 2,00 | 3,00 |
| Zinkoxid | 4,00 | |
| Phenylbenzimidazol Sulfonsäure | 0,50 | |
| Silica Dimethyl Silylat | 0,50 | |
| Sodium Chlorid | 1,00 | 1,00 |
| Citronensäure | 0,086 | |
| Sodium Citrat | 0,174 | |
| Glycerin | | 10,00 |
| Butyleneglycol | 7,50 | |
| Trisodium EDTA | 0,50 | |
| Methylparaben | 0,20 | 0,20 |
| Phenoxyethanol | 0,50 | 0,50 |
| Hexamidine Diisethionat | 0,08 | 0,08 |
| Parfüm | 0,40 | 0,50 |
| Wasser | Ad 100 | Ad 100 |

| | O/W Pick-Cr. 19 |
|---|---|
| Octyldodecanol | 3,00 |
| Caprylic/Capric Triglycerid | 3,00 |
| C12-15- Alkyl Benzoat | 3,00 |
| PVP/Hexadecene Copolymer | 0,50 |
| Sodium Carbomer | 0,10 |
| Xanthan Gum | 0,10 |
| Glyceryl Stearat Citrat | 1,00 |
| Cyclomethicon | 2,00 |
| Vitamin E-Acetat | 0,50 |
| Aminobenzophenon | 2,50 |
| Ethylhexylmethoxycinnamat | 5,00 |
| Ethylhexyltriazon | 1,50 |
| Diethylhexyl Butamido Triazon | 0,50 |
| Titandioxid + Alumina + Simethicone + Aqua | 0,60 |
| Phenyl Dibenzimidazol Tetrasulfonsäure | 0,50 |
| Phenylbenzimidazol Sulfonsäure | 1,50 |
| Distarch Phospaht | 6,00 |
| Silica | 0,60 |
| Citronensäure | 0,086 |
| Sodium Citrat | 0,174 |
| Glycerin | 4,00 |
| Butyleneglycol | 4,00 |
| Octoxyglycerin | 0,30 |
| Trisodium EDTA | 1,00 |
| Glucosylrutin | 0,50 |
| Methylparaben | 0,20 |
| Phenoxyethanol | 0,30 |
| Iodopropynyl Butylcarbamat | 0,18 |
| Etahnol | 5,00 |
| Parfüm | 0,30 |
| Wasser | Ad 100 |

| | O/W-Pick-Cr. 20 |
|---|---|
| Octyldodecanol | 19,00 |
| C12-15- Alkyl Benzoat | 19,00 |
| Dicaprylyl Ether | 8,00 |
| Hydrogenated Polyisobuten | 7,00 |
| Disteardimonium Hectorit | 0,50 |
| Xanthan Gum | 0,20 |
| Phenyltrimethicon | 2,00 |
| Vitamin E-Acetat | 1,00 |
| Aminobenzophenon | 3,00 |
| Octocrylen | 5,00 |
| Bis-Ethylhexyloxyphenol Methoxyphenyl Triazin | 1,50 |
| Titandioxid + Alumina + Simethicone + Aqua | 1,00 |
| Zinkoxid | 2,00 |
| Corn Stärke Modified | 1,00 |
| Glycerin | 7,50 |
| Trisodium EDTA | 0,50 |
| Ubiquinon | 0,25 |
| Phenoxyethanol | 0,50 |
| Diazolidinyl Urea | 0,28 |
| Wasser | Ad 100 |

| | W/O Lotion 21 |
|---|---|
| C12-15- Alkyl Benzoat | 7,50 |
| Butylen Glycol Caprylat/Caprat | 7,50 |
| Dicaprylyl Ether | 7.50 |
| Hydroxyoctacosanyl Hydroxystearat | 2,00 |
| PVP/Hexadecene Copolymer | 0,75 |
| Disteardimonium Hectorit | 0,50 |
| Cetyl PEG/PPG-10/1 Dimethicon | 0,75 |
| Shea Butter | 0,50 |
| Cyclomethicon | 2,00 |
| Vitamin E-Acetat | 0,50 |
| Aminobenzophenon | 1,00 |
| Butyl Methoxydibenzoylmethan | 1,00 |
| Methylen Bis-Benzotriazolyl Tetramethylbutylphenol | 1,00 |
| Titandioxid + Alumina + Simethicone + Aqua | 0,50 |
| Terephthaliden Dicampher Sulfonsäure | 2,00 |
| Bornitrid | 2,00 |
| Zink Sulfat | 1,40 |
| Glycin | 1,50 |
| Glycerin | 10,00 |
| Trisodium EDTA | 1,00 |
| Glucosylrutin | 0,50 |
| Phenoxyethanol | 0,40 |
| DMDM Hydantoin | 0,60 |
| Etahnol | 2,00 |
| Parfüm | 0,40 |
| Wasser | Ad 100 |

| | Pick-Lotion 22 |
|---|---|
| Mineralöl | 16,00 |
| Octyldodecanol | 5,00 |
| Caprylic/Capric Triglycerid | 6,00 |
| Hydroxyoctacosanyl Hydroxystearat | 1,50 |
| PVP/Hexadecene Copolymer | 1,00 |
| Disteardimonium Hectorit | 1,00 |
| Cyclomethicon | 2,00 |
| Aminobenzophenon | 1,00 |
| Butyl Methoxydibenzoylmethan | 1,00 |
| Methylbenzylidene Campher | 2,00 |
| Drometritriazol Trisiloxan | 2,00 |
| Titandioxid + Alumina + Simethicone + Aqua | 2,00 |
| Zinkoxid | 2,00 |
| Silica Dimethyl Silylat | 0,50 |
| Magnesium Sulfat | 0,70 |
| Glycerin | 3,00 |
| Butyleneglycol | 5,00 |
| Trisodium EDTA | 1,00 |
| Porpylen Carbonat | 0,33 |
| Methylparaben | 0,21 |
| Propylparaben | 0,07 |
| Phenoxyethanol | 0,50 |
| Wasser | Ad 100 |

**Patentansprüche**

**1.** Pickering-Emulsionen, welche feindisperse Systeme vom Typ Wasser-in-Öl oder Öl-in-Wasser darstellen, enthaltend

(1) eine Ölphase,
(2) eine Wasserphase,
(3) mindestens einen Typ mikrofeiner Partikel, die

a) eine mittlere Partikelgröße von weniger als 200 nm haben, die
b) sowohl hydrophile als auch lipophile Eigenschaften zeigen, welche also amphiphilen Charakter besitzen und sowohl in Wasser als auch in Öl dispergierbar sind und die

c) gegebenenfalls oberflächlich beschichtet sind,

(4) mindestens ein Hydroxybenzophenon und
(5) höchstens 1 Gew.-% eines oder mehrerer Emulgatoren.

**2.** Pickering-Emulsionen nach Anspruch 1, **dadurch gekennzeichnet, daß** sie emulgatorfrei sind.

**3.** Pickering-Emulsionen nach einem der vorhergehenden Ansprüche, **dadurch gekennzeichnet, daß** der Gehalt der verwendeten Partikel zwischen 0,1 Gew.-% und 30 Gew.-% ist, bezogen auf das Gesamtgewicht der Zubereitungen.

**4.** Pickering-Emulsionen nach einem der vorhergehenden Ansprüche, **dadurch gekennzeichnet, daß** der Partikeldurchmesser der verwendeten Partikel zwischen 5 nm und 100 nm liegt.

**5.** Pickering-Emulsionen nach einem der vorhergehenden Ansprüche, **dadurch gekennzeichnet, daß** die verwendeten Partikel gewählt werden aus der Gruppe bestehend aus amphiphilen Metalloxiden, Bornitrid, modifizierten Schichtsilikaten, mikrofeinen Polymerpartikeln und modifizierten Polysacchariden, wobei die Partikel sowohl einzeln als auch im Gemisch vorliegen können.

**6.** Pickering-Emulsionen nach einem der vorhergehenden Ansprüche, **dadurch gekennzeichnet, daß** die verwendeten Partikel oberflächlich wasserabweisend behandelt sind, wobei der amphiphile Charakter der Partikel gebildet wird bzw. erhalten bleibt.

**7.** Pickering-Emulsionen nach einem der vorhergehenden Ansprüche, **dadurch gekennzeichnet, daß** sie weitere Hilfsstoffe enthalten, die zur Verminderung oder Verhinderung eines stumpfen oder trockenen Hautgefühls nach deren Auftragen beitragen können.

**8.** Pickering-Emulsionen nach einem der vorhergehenden Ansprüche, **dadurch gekennzeichnet, daß** als Hydroxybenzophenon der 2-(4'-Diethylamino-2'hydoxybenzoyl)-benzoesäurehexylester, welcher durch die chemische Strukturformel

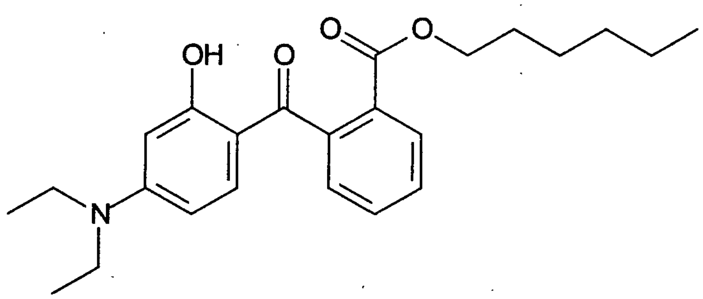

gekennzeichnet ist, gewählt wird.

**9.** Pickering-Emulsionen nach einem der vorhergehenden Ansprüche, **dadurch gekennzeichnet, daß** sie mindestens eine weitere UV-Filtersubstanz, gewählt aus der Gruppe Triazine, Benzotriazole, der bei Raumtemperatur flüssigen UV-Filter und organische und/oder anorganische Pigmente, enthält.

**10.** Pickering-Emulsionen nach einem der vorhergehenden Ansprüche, **dadurch gekennzeichnet, daß** sie mindestens eine weitere UV-A-Filtersubstanz und/oder einen Breitbandfilter, gewählt aus der Gruppe Dibenzoylmethanderivate [insbesondere das 4-(tert.-Butyl)-4'-methoxydibenzoylmethan], Phenylen-1,4-bis-(2-benzimidazyl)-3,3'-5,5'-tetrasulfonsäure-bis-natriumsalz, 1,4-di(2-oxo-10-Sulfo-3-bornylidenmethyl)-Benzol und dessen Salze und 2,4-Bis-{[4-(2-Ethyl-hexyloxy)-2-hydroxy]-phenyl}-6-(4-methoxyphenyl)-1,3,5-triazin, enthält, wobei die weiteren Filtersubstanzen jeweils einzeln oder in beliebigen Kombinationen miteinander vorliegen können.

**11.** Pickering-Emulsionen nach einem der vorhergehenden Ansprüche, **dadurch gekennzeichnet, daß** sie mindestens ein Flavonglycosid, insbesondere α-Glucosylrutin, und/oder Vitamin E und/oder dessen Derivate enthält.

**12.** Verwendung von Stoffkombinationen enthaltend

(1) eine Ölphase,
(2)eine Wasserphase,
(3)mindestens einen Typ mikrofeiner Partikel, die

a) eine mittlere Partikelgröße von weniger als 200 nm haben, die
b) sowohl hydrophile als auch lipophile Eigenschaften zeigen, welche also amphiphilen Charakter besitzen und sowohl in Wasser als auch in Öl dispergierbar sind und die
c) gegebenenfalls oberflächlich beschichtet sind,

(4)mindestens ein Hydroxybenzophenon und
(5)höchstens 1 Gew.-% eines oder mehrerer Emulgatoren.

zur Herstellung von Zubereitungen zur Prophylaxe und Behandlung kosmetischer oder dermatologischer Hautveränderungen, wie sie bei der Hautalterung auftreten.